Europäisches Patentamt

European Patent Office

Office européen des brevets

(19)

(11) Numéro de publication: **0 105 788**

A1

(12) **DEMANDE DE BREVET EUROPEEN**

(21) Numéro de dépôt: 83401859.0

(22) Date de dépôt: 23.09.83

(51) Int. Cl.³: **C 02 F 3/28**
C 02 F 3/10, B 01 J 8/08

(30) Priorité: 28.09.82 FR 8216273

(43) Date de publication de la demande:
18.04.84 Bulletin 84/16

(84) Etats contractants désignés:
BE DE GB IT

(71) Demandeur: "DEGREMONT" Société dite:
183, Avenue du 18 Juin 1940
F-92508 Rueil-Malmaison Cédex(FR)

(72) Inventeur: Rovel, Jean-Marie
74, Rue Sophie Rodrigues
F-92500 Rueil Malmaison(FR)

(72) Inventeur: Prevot, Claude
Rue du Belvédère de la Ronce
F-92410 Ville d'Avray(FR)

(72) Inventeur: Nicol, Roger
19, rue Jean Jaurès
F-92166 Vanves(FR)

(74) Mandataire: Armengaud, Charles
Cabinet ARMENGAUD AINE 3, Avenue Bugeaud
F-75116 Paris(FR)

(54) Procédé et appareil de traitement anaérobie d'eaux résiduaires dans un filtre à remplissage de matériau granulaire.

(57) Procédé de traitement anaérobie d'eaux résiduaires dans un filtre à remplissage de matériau granulaire support de la biomasse formée, avec production simultanée de biogaz, suivant lequel l'eau à traiter et le matériau supportant la biomasse sont amenés à circuler en continu au moyen d'un système de pompage utilisant comme fluide moteur une partie du biogaz produit au cours du traitement anaérobie. L'appareil pour la mise en oeuvre du procédé, est constitué par une enceinte (1) fermée contenant un remplissage de matériau granulaire (2) et il est muni, comme système de pompage, d'au moins un tube de pompe-mammouth à gaz (3) utilisant comme fluide moteur une partie du biogaz produit au cours du traitement.

EP 0 105 788 A1

./...

Croydon Printing Company Ltd

- 1 -

<u>Procédé et appareil de traitement anaérobie d'eaux résiduaires</u>
<u>dans un filtre à remplissage de matériau granulaire</u>

L'invention concerne un procédé de traitement anaérobie d'eaux résiduaires urbaines ou industrielles avec production simultanée de biogaz ;
elle concerne plus particulièrement un procédé de traitement dans lequel les eaux résiduaires sont soumises à l'action de microorganismes
anaérobies dans un filtre contenant un remplissage de matériau granulaire sur lequel se fixe la biomasse formée. L'invention concerne également l'appareil pour la mise en oeuvre du procédé.

De nombreux procédés de ce type ont déjà été décrits dans lesquels
l'eau résiduaire à traiter est amenée à traverser de bas en haut ou de
haut en bas un filtre anaérobie à remplissage d'un matériau support
fixe ou mobile. On connaît plusieurs types de procédés dans lesquels le
matériau support est fixe : le matériau peut être émergé et traversé de
haut en bas par l'eau à traiter ; les temps de séjour sont alors très
courts et nécessitent un taux de recyclage élevé. Le matériau support
peut être immergé, l'eau à traiter circulant de bas en haut ou de haut
en bas. Dans le cas où l'on utilise un matériau ordonné, matière plastique par exemple, celui-ci présente une surface spécifique faible, donc
ne permet la fixation que d'une faible quantité de biomasse ; en outre se
posent des problèmes de répartition de l'eau à traiter et de l'eau recyclée. Dans le cas où l'on utilise un matériau en vrac ou granulaire
(argile, silex, ...) il existe des risques importants de colmatage du
filtre, la biomasse ayant tendance à croître et à bloquer les passages libres pour l'eau, la vitesse de passage de l'eau étant trop faible pour arracher la biomasse en excès.

On connaît également des procédés dans lesquels le matériau support est mobile, expansé ou fluidisé par exemple par l'eau à traiter et l'eau recyclée ; il y a alors des risques de colmatage des dispositifs d'introduction de ces fluides et il se pose des problèmes importants de répartition de l'eau à traiter et de l'eau recyclée. De plus, certains de ces procédés connus nécessitent une décantation de l'eau traitée à l'extérieur du filtre lui-même.

L'invention pallie ces inconvénients et permet en outre un réglage simple du procédé.

La présente invention consiste en un procédé de traitement anaérobie d'eaux résiduaires dans un filtre à remplissage de matériau granulaire support de la biomasse formée, avec production simultanée de biogaz, caractérisé en ce que l'eau à traiter et le matériau supportant la biomasse sont amenés à circuler en continu au moyen d'un système de pompage du type "Béduwé" utilisant comme fluide moteur une partie du biogaz produit au cours du traitement anaérobie.

Le système de pompage d'un fluide par tube "Béduwé" est bien connu, dans lequel l'insufflation d'un gaz sous pression dans un tube permet l'aspiration du fluide par ce tube. Dans le procédé suivant l'invention le biogaz servant de fluide moteur au système de pompage est surpressé avant son utilisation. Son débit est régulé en fonction de la vitesse de circulation désirée du matériau. En effet, la quantité de matériau mis en turbulence est fonction de la variation de densité du matériau granulaire entre la phase de démarrage où l'ensemencement n'est pas encore effectif et le régime stationnaire où le matériau est enrobé de biomasse. Le réglage simple et précis du débit de gaz permet d'obtenir une turbulence constante ainsi qu'une quantité optimale de matériau en circulation par unité de volume de réacteur.

Une autre caractéristique du procédé est que l'eau à traiter est séparée du matériau supportant la biomasse dans une zone de décantation située à la partie supérieure de la zone de turbulence créée par le système de pompage, le matériau supportant la biomasse entrant dans cette zone de décantation, retombant gravitairement dans la zone de turbulence et l'eau traitée décantée étant extraite et éventuellement recyclée.

Le matériau granulaire utilisé : pierre ponce, argile expansée, charbon actif, matières plastiques, par exemple, est constitué de particules de diamètre de préférence inférieur à 1 mm, offrant une bonne surface spécifique et une bonne résistance à l'abrasion.

La densité du matériau est telle que la différence entre la densité du matériau enrobé de biomasse et celle de l'eau à traiter est inférieure à 0,3 et de préférence comprise entre 0,05 et 0,15.

Le procédé, comme tous les procédés de traitement anaérobie, est avantageusement mis en oeuvre à une température de 35°C.

L'appareil pour mettre en oeuvre le procédé suivant l'invention, constitué par une enceinte fermée contenant un remplissage de matériau granulaire, est caractérisé en ce qu'il est muni d'un système de pompage du type "Béduwé", avantageusement constitué par au moins un tube "Béduwé" disposé verticalement dans cette enceinte. Dans une forme de réalisation de l'invention l'appareil comporte quatre zones superposées : une zone inférieure de tassement du matériau et de reprise de ce matériau et de l'eau à traiter par le système de pompage alimenté en gaz moteur ; une zone de turbulence dans laquelle circulent en continu l'eau à traiter et le matériau granulaire supportant la biomasse et dans la-

0105788

- 4 -

quelle est introduite l'eau à traiter ; une zone de décantation d'où est extraite l'eau traitée, cette zone étant séparée de la zone de turbulence par une cheminée entourant la partie supérieure d'un tube "Béduwé" et comportant à sa base une partie évasée coiffant la zone de turbulence ; une zone de stockage du gaz produit, d'où est extrait le biogaz dont une partie est insufflée dans le tube après avoir été surpressée.

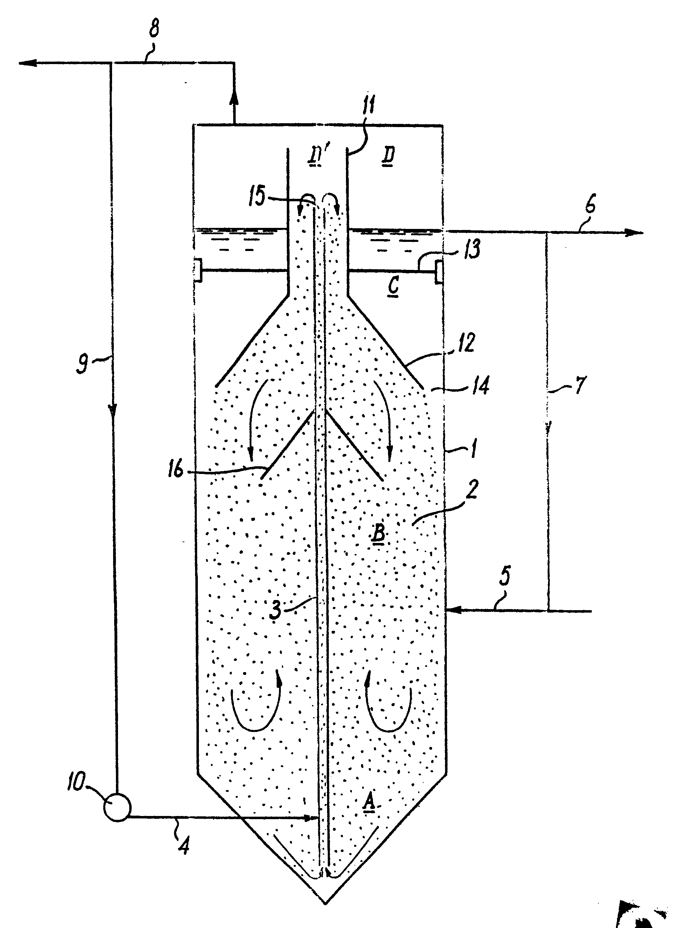

La description qui va suivre en référence à la figure unique du dessin annexé permettra de mieux comprendre l'invention. Il est bien précisé qu'il s'agit uniquement d'un exemple, non limitatif, de réalisation de l'invention.

Dans le cas traité l'appareil, suivant l'invention, comporte un seul tube "Béduwé". Cependant, un appareil suivant l'invention peut comporter plusieurs tubes "Béduwé" ainsi que plusieurs cheminées séparant les zones de turbulence des zones de décantation.

L'appareil est constitué par une enceinte fermée 1 contenant un remplissage de matériau granulaire 2 et dans laquelle est disposé un tube "Béduwé" 3 alimenté en gaz moteur par la canalisation 4. L'eau à traiter est introduite dans l'enceinte par une canalisation 5. L'eau traitée est extraite par une canalisation 6 et éventuellement en partie recyclée par une canalisation 7. Le biogaz produit est extrait à la partie supérieure de l'enceinte par une canalisation 8 et en partie réutilisé pour l'alimentation du tube 3. La quantité nécessaire de biogaz est amenée par une canalisation 9 à un surpresseur 10 pour être injectée dans le système de pompage.

L'appareil présente quatre zones superposées :

- une zone A de tassement du matériau avec reprise par le tube 3 constituée par le fond de l'appareil, de préférence de forme conique au-dessus de ce tube ;

- une zone B de turbulence dans laquelle circulent en continu l'eau à traiter et le matériau granulaire supportant la biomasse et où s'effectue le traitement. La turbulence, donc le bon contact entre biomasse et support granulaire, est maintenue dans la zone considérée par au moins un déflecteur 16 prévu sur le tube 3 ;

- une zone C de décantation délimitée au-dessus de la zone de turbulence par une cheminée 11 entourant la partie supérieure du tube 3 et dont la base est constituée par une paroi évasée 12 coiffant la zone de turbulence. Un dispositif de fixation 13 réglable permet de faire varier la position de la cheminée dans le réacteur suivant la qualité de l'eau à traiter ; de cette zone est extraite l'eau traitée tandis que le matériau supportant la biomasse retombe gravitairement dans la zone de turbulence par l'espace 14 compris entre la paroi inférieure de la cheminée et la paroi interne de l'enceinte 1 ;

- une zone D de stockage du biogaz produit au cours du traitement, zone située à la partie supérieure de l'appareil au-dessus du niveau liquide ; le dégazage de l'eau s'effectue à la sortie 15 du tube 3 dans une zone D' délimitée par la partie supérieure de la cheminée. Le gaz produit est extrait par la canalisation 8.

### Exemple

On a réalisé, pour mettre en oeuvre le procédé suivant l'invention, un appareil de 5 m de hauteur totale comportant une zone A de tassement du matériau de 0,5m de hauteur, une zone B de turbulence de 3 m de hauteur et une zone C de décantation de 1 m de hauteur.

La surface de l'appareil était de 2 m$^2$.

La base de la zone de tassement de forme conique avait un angle au sommet de 45°.

On a traité dans cet appareil une eau résiduaire à pollution essentiellement soluble et biodégradable de DCO moyenne 4 g/l introduite à un

débit de 1,5 m$^3$/h. Le débit du gaz moteur insufflé dans le système de pompage était de 6 m$^3$/h puis a été réglé à 4 m$^3$/h.

On a obtenu, après une mise en équilibre, une production de biogaz contenant de 60 à 70 % de méthane de 2,5 à 3 Nm$^3$/h et une eau traitée à 600 mg/l de DCO et 300 mg/l de matières en suspension.

- 1 -

Revendications de brevet

1. Procédé de traitement anaérobie d'eaux résiduaires dans un filtre à remplissage de matériau granulaire support de la biomasse formée, avec production simultanée de biogaz, caractérisé en ce que l'eau à traiter et le matériau supportant la biomasse sont amenés à circuler en continu au moyen d'un système de pompage utilisant comme fluide moteur une partie du biogaz produit au cours du traitement anaérobie.

2. Procédé suivant la revendication 1 caractérisé en ce que le biogaz servant de fluide moteur au système de pompage est surpressé avant son utilisation.

3. Procédé suivant l'une quelconque des revendications précédentes caractérisé en ce que le débit de biogaz utilisé comme fluide moteur du système de pompage est régulé en fonction de la vitesse de circulation désirée du matériau granulaire et de l'eau à traiter, vitesse fonction de la différence de densité entre le matériau granulaire supportant la biomasse et l'eau à traiter.

4. Procédé suivant l'une quelconque des revendications précédentes caractérisé en ce que la différence entre la densité du matériau granulaire supportant la biomasse et celle de l'eau à traiter est inférieure à 0,3.

5. Procédé suivant l'une quelconque des revendications précédentes caractérisé en ce que la différence entre la densité du matériau granulaire enrobé de biomasse et celle de l'eau à traiter est comprise entre 0,05 et 0,15.

6. Procédé suivant l'une quelconque des revendications précédentes caractérisé en ce que les particules de matériau granulaire ont un diamètre inférieur à 1 mm.

7. Appareil pour la mise en oeuvre du procédé suivant l'une quelconque des revendications précédentes, constitué par une enceinte fermée contenant un remplissage de matériau granulaire caractérisé en ce qu'il est muni d'un système de pompage du type "Béduwé" utilisant comme fluide moteur une partie du biogaz produit au cours du traitement anaérobie.

8. Appareil suivant la revendication 7 caractérisé en ce qu'il présente quatre zone superposées :
- une zone inférieure (A) de tassement du matériau et de reprise de ce matériau et de l'eau à traiter par le système de pompage ;
- une zone (B) de turbulence dans laquelle circulent en continu l'eau à traiter et le matériau supportant la biomasse ;
- une zone (C) de décantation d'où est extraite l'eau traitée ;
- une zone (D) de stockage du biogaz produit dont une partie est insufflée dans le système de pompage après avoir été surpressée.

9. Appareil suivant l'une quelconque des revendications 7 et 8 carécté-risé en ce que le système de pompage qu'il comporte consiste en au moins une tube "Béduwé" (3) dans lequel circulent en continu l'eau à traiter et le matériau granulaire, sous la pression/du biogaz produit au d'une partie cours du traitement.

10. Appareil suivant l'une quelconque des revendications 7 à 9 caractérisé en ce que la zone (C) de décantation est délimitée par une cheminée (11) entourant chaque tube (3) et comportant une base évasée (12) coiffant la zone de turbulence (B).

11. Appareil suivant l'une quelconque des revendications 7 à 10 caractérisé en ce que le tube (3) est muni d'au moins un déflecteur (16) pour favoriser la turbulence dans la zone (B).

**Office européen des brevets**

## RAPPORT DE RECHERCHE EUROPEENNE

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| Y | US-A-4 111 808 (J.H. FAIR)<br><br>* Colonne 2, ligne 27 - colonne 3, ligne 29; colonne 4, lignes 14-35 *<br><br>--- | 1,2,7, 9 | C 02 F 3/28<br>C 02 F 3/10<br>B 01 J 8/08 |
| Y | GB-A-2 014 555 (CHIYODA CHEMICAL)<br>* Page 4, lignes 74-106 *<br><br>--- | 1,2,7, 9 | |
| Y | EP-A-0 026 163 (DOMTAR)<br><br>* Page 3, ligne 13 - page 5, ligne 12 *<br><br>--- | 1,3,7, 8,9 | |
| A | FR-A-2 189 328 (ECOLOTROL)<br>* Page 5, lignes 1-19 *<br><br>--- | 4-6 | |
| A | GB-A-1 527 766 (DOWA MINING)<br>* Figure 2 *<br><br>--- | 4-6 | DOMAINES TECHNIQUES RECHERCHES (Int. Cl. 3) |
| A | NL-A- 148 112 (INFILCO)<br><br>* Figure 2 *<br><br>--- | | C 02 F<br>B 01 J |
| A | FR-A-2 446 259 (DEGREMONT)<br><br>* Page 5, ligne 7 - page 6 *<br><br>----- | | |

Le présent rapport de recherche a été établi pour toutes les revendications

| Lieu de la recherche<br>LA HAYE | Date d'achèvement de la recherche<br>03-01-1984 | Examinateur<br>TEPLY J. |
|---|---|---|